# EUROPÄISCHE PATENTSCHRIFT

(11) **EP 3 631 441 B1**
(45) Veröffentlichungstag und Bekanntmachung des Hinweises auf die Patenterteilung: **27.07.2022**
(21) Anmeldenummer: 18726795.0
(22) Anmeldetag: 23.05.2018
(51) Int. Cl.: G01N 33/00, A61M 16/00, G01N 1/22

(54) **ÜBERWACHUNGSEINRICHTUNG FÜR EINE ANLAGE ZUR ERZEUGUNG MEDIZINISCHER DRUCKLUFT**
MONITORING DEVICE FOR A SYSTEM FOR GENERATING MEDICAL PRESSURISED AIR
DISPOSITIF DE SURVEILLANCE D'UNE INSTALLATION POUR LA PRODUCTION D'AIR COMPRIMÉ MÉDICAL

(30) Priorität: 24.05.2017 DE 102017005011
(43) Veröffentlichungstag der Anmeldung: 08.04.2020
(73) Patentinhaber: Drägerwerk AG & Co. KGaA, 23558 Lübeck (DE)
(72) Erfinder: HARM, Reiner, 23683 Scharbeutz (DE); MARTENSEN, Kjer, 23558 Lübeck (DE); LOSCH, Matthias, 23556 Lübeck (DE); BARTEN, Ronny, 23562 Lübeck (DE); REINCKE, Stefan, 20099 Hamburg (DE)
(86) Internationale Anmeldenummer: PCT/EP2018/063438
(87) Internationale Veröffentlichungsnummer: WO 2018/215505

(56) Entgegenhaltungen:
- DE-A1-102010 014 222
- DE-B3-102012 018 697
- US-B1- 6 266 995

## Beschreibung

Die Erfindung betrifft eine Einrichtung zur Überwachung einer Anlage zur Erzeugung medizinischer Druckluft. Die Druckluft wird der Druckluftanlage stromabwärts einer Druckluftkonditionierungseinheit entnommen und über eine Messluftleitung der Überwachungseinrichtung zugeführt. Die Überwachungseinrichtung verfügt über wenigstens einen Sensor, der in Abhängigkeit einer Eigenschaft der durch die Messluftleitung geförderten Druckluft ein Messsignal erzeugt. Dieses Messsignal wird ausgewertet und eine Information über die Druckluft mittels einer Ausgabeeinheit einem Nutzer zur Verfügung gestellt.

In Krankenhäusern werden Drucklufterzeugungsanlagen eingesetzt, um durch ein Netz an Druckluftversorgungsleitungen verschiedene Druckluftverbraucher innerhalb des Krankenhauses mit medizinischer Druckluft zu versorgen. Hierbei wird die von der Drucklufterzeugungsanlage erzeugte medizinische Druckluft von einer Druckluftüberwachungseinrichtung auf verschiedene Parameter, insbesondere die Konzentration relevanter Gase, untersucht. Auf diese Weise werden beispielsweise Fremdgaskonzentrationen an Schwefeldioxid oder Stickstoffdioxid erfasst, um sicherzustellen, dass den Druckluftverbrauchern eine medizinisch einwandfreie medizinische Druckluft zur Verfügung gestellt werden kann.

Für die erforderlichen Messungen werden in der Druckluftüberwachungseinrichtung Sensoren zur Erfassung der relevanten Parameter der medizinischen Druckluft eingesetzt. Zu berücksichtigen ist, dass medizinische Druckluft als Arzneimittel dem europäischen Arzneibuch unterliegt, sodass entsprechend hohe Anforderungen an die Qualität dieser Druckluft gestellt werden, die in DIN EN ISO 7396-1 aufgelistet sind. Gemäß dieser Norm dürfen vorgegebene Konzentration an Kohlendioxid, Kohlenmonoxid, Öl, Schwefeldioxid, Stickstoffoxid nicht überschritten werden. Weiterhin muss Sauerstoff mit einem Anteil von 20,4 - 21,4 Vol% in der Druckluft vorhanden sein. Um die zuvor beschriebenen Anforderungen an die medizinische Druckluft erfüllen zu können, wird diese mittels einer Aufbereitungseinrichtung mit Filtern gereinigt und durch eine Entfeuchtungseinrichtungen entfeuchtet, wobei der Feuchtegehalt weniger als 67 ppm beträgt.

Neben der dauerhaften Überwachung der Druckluft ist es ferner erforderlich, in regelmäßigen Abständen Druckluftproben der Druckluftanlage zu entnehmen, diese zu untersuchen umso feststellen zu können, ob die Druckluftüberwachungsanlage einwandfrei funktioniert. Hierfür sind üblicherweise spezielle Zapfstellen vorgesehen, die eine entsprechende Entnahme von Druckluft aus der Druckluftanlage ermöglichen.

Aus der DE 10 2010 014 222 A1 ist eine Druckluftüberwachungseinrichtung bekannt, die die mithilfe einer Druckluftkonditionierungsanlage erzeugte Druckluft hinsichtlich ihrer Eigenschaften und unter Berücksichtigung der gesetzlichen Vorschriften dauerhaft überwacht. Als wesentliches Bauteil verfügt die beschriebene Druckluftüberwachungseinrichtung über eine Luftbefeuchtungseinheit, die die den Sensoren über eine Messleitung zugeführte Druckluft befeuchtet, sodass ein Austrocknen der Sensoren zuverlässig vermieden wird. Bevorzugt verfügt der Luftbefeuchter hierbei über eine semipermeable wasserdurchlässige Membran, die keine Schadgase, wie etwa Kohlenmonoxid, Schwefeldioxid oder Stickstoffdioxid absorbiert und außerdem auch bei einem Überdruck auf einer Seite der Membran keine Schadgase zur anderen Seite hindurchdiffundieren lässt.

In DE 10 2012 018 697 B3 wird eine zentrale Gasversorgungsanlage für medizinische Druckluft beschrieben. Stromabwärts von einem Puffervolumen 5 sind ein Vorfilter 7 und danach eine Gasaufbereitung 8 angeordnet. Zwischen dem Vorfilter 7 und der Gasaufbereitung 8 ist ein Anschluss 11 zur Entnahme einer Gasprobe angeordnet, an welchem ein Messgerät 12 zur Gasanalyse angeschlossen ist. Dadurch werden Verunreinigungen bereits stromaufwärts von der Gasaufbereitung 8 erkannt.

In US 6266995 B1 wird ein tragbares Testgerät beschrieben, mit welchem sich medizinisches Gas testen lässt. Dieses Testgerät lässt sich mit verschiedenen Komponenten einer Versorgungseinrichtung verbinden und umfasst einen Gassensor, einen Druck-Transducer und einen Sauerstoff-Transducer.

Ausgehend von dem vorgenannten bekannten Stand der Technik liegt der Erfindung die Aufgabe zugrunde, eine Vorrichtung zur Überwachung einer Anlage zur Versorgung von Verbrauchern mit medizinischer Druckluft dahingehend weiterzubilden, dass mit verhältnismäßig geringem technischen Aufwand und somit verhältnismäßig kostengünstig eine Überwachung der erzeugten medizinischen Druckluft im Rahmen der gesetzlichen Vorschriften ermöglicht wird. Gleichzeitig soll eine Entnahme von Druckluftproben auf einfache Weise möglich sein. Durch die anzugebende Druckluftüberwachungseinrichtung sollte weiterhin eine kompakte Anordnung der Sensoren und Komponenten zu realisieren sein und eine vorteilhafte technische Lösung zur Überwachung der Konzentration von Kohlenstoffmonoxid in der Druckluft angegeben werden.

Die vorstehende Aufgabe wird mithilfe einer Druckluftüberwachungseinrichtung gemäß Anspruch 1 gelöst. Vorteilhafte Ausführungsformen der Erfindung sind Gegenstand der abhängigen Ansprüche und werden in der folgenden Beschreibung unter teilweiser Bezugnahme auf die Figuren näher erläutert.

Die Erfindung betrifft eine Druckluftüberwachungseinrichtung für eine Anlage zur Erzeugung medizinischer Druckluft mit einer Messluftleitung zur Entnahme von Druckluft aus einer Druckluftversorgungsleitung stromabwärts einer Druckluftkonditionierungseinheit. Die Druckluftüberwachungseinrichtung verfügt über wenigstens einen Sensor zur Erzeugung eines Messsignals in Abhängigkeit einer Eigenschaft der durch die Messluftleitung geförderten Druckluft, über wenigstens einen Luftbefeuchter zur Befeuchtung der Druckluft stromaufwärts des Sensors und über wenigstens eine Ausgabeeinheit, die unter Zugrundelegung des Messsignals eine Information über die Eigenschaft der Druckluft an einem Nutzer ausgibt. Die erfindungsgemäße Druckluftüberwachungseinrichtung zeichnet sich dadurch aus, dass sie eine Zapfstelle zur Entnahme von Druckluft und ein Stellorgan zur Einstellung eines in der Messluftleitung herrschenden Volumen- und/oder Massenstroms umfasst, wobei das Stellorgan in einem Messmodus in die Zapfstelle eingesteckt und in einem Druckluftentnahmemodus aus der Zapfstelle entnommen ist. Das wesentliche technische Merkmal der vorliegenden Erfindung beruht somit darauf, dass eine herkömmliche Zapfstelle, wie sie auch zum Anschluss von Druckluftverbrauchern verwendet wird, einerseits zur Entnahme einer Druckluftprobe verwendet wird und andererseits ein Stellorgan (Flowmeter) in die Zapfstelle bzw. den Druckluftanschluss eingesteckt wird. Auf verhältnismäßig einfache Weise ist es somit während des Betriebs der Druckluftüberwachungseinrichtung möglich, zwischen einem Messmodus, in dem eine Volumen- und/oder Massenstrommessung durchgeführt wird und einem Druckluftentnahmemodus, in dem der Druckluftanlage über die Zapfstelle eine Druckluftprobe entnommen wird, zu wechseln. Ebenso ist es grundsätzlich denkbar, die Zapfstelle mit ihrer Kupplung zum Anschluss eines Druckluftverbrauchers zu nutzen, sobald sich das Stellorgan nicht im eingesteckten Zustand befindet.

Auf bevorzugte Weise ist das Stellorgan derart ausgeführt, dass unterschiedliche diskrete Sollwerte für den in der Messluftleitung herrschenden Volumen- und/oder Massenstrom einstellbar sind. Vorzugsweise ist das Stellorgan derart ausgeführt, dass in der Messluftleitung Volumenstromwerte von 0 l/min, 0,1 l/min, 0,2 l/min, 0,3 l/min, 0,4 l/min, 0,5 l/min, 0,6 l/min, 0,8 l/min oder 1 l/min eingestellt werden können. Besonders vorteilhaft ist eine Ausführung des Stellorgans, bei der zumindest Sollwerte für den Volumenstrom von 0,1 l/min oder 0,2 l/min eingestellt werden.

Gemäß einer speziellen Ausführungsform verfügt das Stellorgan über wenigstens eine Blende, die eine Verstellung des Volumenstroms bewirkt. Das Stellorgan kann manuell, beispielsweise durch Vorsehen eines Schalters, der in unterschiedlichen Positionen einrastet, oder automatisiert betätigbar sein. Generell ist es denkbar, dass das Stellorgan über eine geeignete Schnittstelle von einem externen Gerät ansteuerbar ist und sogar, dass bei Bedarf Daten uni- oder bidirektional zwischen dem Stellorgan und einem externen Gerät austauschbar sind. im Übrigen ist es von Vorteil, wenn das Stellorgan über eine Anzeigeeinheit verfügt, über die wenigstens ein Betriebsparameter, insbesondere der eingestellte Sollwert des in der Messluftleitung herrschenden Volumen- und/oder Massenstroms, zumindest zeitweise ausgegeben wird. Auf geeignete Weise verfügt das Stellorgan bei Bedarf über eine eigene Versorgungseinheit mit Batterie oder Akkumulator zur Versorgung mit elektrischer Energie oder ist über eine externe Stromversorgung mit elektrischer Energie zu versorgen.

Gemäß einer besonderen Ausführungsform der Erfindung ist die Zapfstelle stromabwärts der Druckluftkonditionierungseinheit und stromaufwärts der Luftbefeuchtungsanlage, die die Druckluft befeuchtet bevor diese den Sensoren zugeführt wird, angeordnet. Auf diese Weise wird sichergestellt, dass eine Druckluftprobe entnommen werden kann, die über die in der Druckluftversorgungsanlage ebenfalls vorhandenen Parameter verfügt.

Auf vorteilhafte Weise ist das Stellorgan zur Einstellung des in der Messleitung herrschenden Volumen- und/oder Massenstroms zerstörungsfrei und werkzeugfrei in die Zapfstelle einsteckbar und aus der Zapfstelle entnehmbar. In diesem Zusammenhang soll deutlich zum Ausdruck gebracht werden, dass es vorteilhaft ist, wenn das entsprechende Stellorgan verhältnismäßig einfach in die Zapfstelle, die vorzugsweise als herkömmliche Druckluftkupplung ausgeführt ist, eingesteckt und auch wieder entnehmbar ist.

Gemäß einer besonderen Weiterbildung der Erfindung ist der wenigstens eine Sensor zur Erfassung einer Eigenschaft der Druckluft als elektrochemischer Sensor ausgeführt. Ein derartiger Sensor zeichnet sich dadurch aus, dass er in Abhängigkeit der Gaskonzentration eines spezifischen Gases in der gemessenen Druckluft einen Messstrom erzeugt, welcher als Messsignal vorliegt und mit einer geeigneten Auswerteeinheit ausgewertet wird. Das so erhaltene Messsignal wird schließlich einer Ausgabeeinheit zugeführt, die eine Information über die gemessene Eigenschaft der Druckluft, insbesondere die Konzentration des spezifischen Gases, an einen Nutzer ausgibt. Auf besonders vorteilhafte Weise ist zumindest ein Sensor vorgesehen, der die Konzentration von Kohlenstoffmonoxid (CO) innerhalb der erzeugten medizinischen Druckluft erfasst. Wesentlich hierbei ist, dass die CO-Konzentration innerhalb der Luft stromabwärts sämtlicher Druckluftkonditionierungseinheiten vorgesehen ist.

Gemäß einer besonderen Weiterbildung ist ferner eine Durchflussröhre vorgesehen, mit der der Volumen- und/oder Massestrom in der Messgasleitung gemessen bzw. überwacht wird. In diesem Zusammenhang ist es generell denkbar, dass der von der Durchflussröhre gemessene Wert verwendet wird, um die Einstellung des Stellorgans zu überwachen und bei Bedarf auf den benötigten Wert einzustellen. In einer sehr speziellen Ausführungsform der Erfindung ist vorgesehen, dass der von der Durchflussröhre ermittelte Wert des momentanen Volumenstroms verwendet wird, um das Stellorgan vorzugsweise automatisiert zu verstellen, zumindest sofern der Momentanwert des Volumenstroms vom Sollwert abweicht.

In einer speziellen Ausführungsform der Erfindung wird mit Hilfe einer erfindungsgemäß ausgeführten Druckluftüberwachungseinrichtung ausschließlich die Kohlenstoffmonoxidkonzentration der Druckluft überwacht. In diesem Fall verfügt die Druckluftüberwachungseinrichtung über einen Sensor zur Erfassung der CO-Konzentration in der Druckluft, über eine Luftbefeuchtungseinheit zur Anfeuchtung der Luft in der Messgasleitung und über eine Zapfstelle mit einem Stellorgan, das in die Zapfstelle eingesteckt oder aus dieser entfernt werden kann.

Bevorzugt sind die vorgenannten Komponenten in einem Gehäuse untergebracht, sodass eine vergleichsweise kompakte Einheit zur Überwachung des CO-Gehaltes eines Druckluftstromes bereitgestellt wird, die gleichzeitig die Möglichkeit bietet, auf einfache Weise eine Druckluftprobe entnehmen zu können. Vorzugsweise ist in dem Gehäuse auch noch eine Durchflussmessröhre zur Messung des Volumenstroms der Druckluft innerhalb der Messluftleitung angeordnet.

Auf vorteilhafte Weise verfügt die erfindungsgemäß ausgeführte Druckluftüberwachungseinrichtung über wenigstens einen Taupunktsensor. Auf diese Weise wird die Luftfeuchte der Druckluft überwacht und sichergestellt, dass der vorgeschriebene Feuchtigkeitsgehalt nicht überschritten wird. Grundsätzlich ist es in diesem Zusammenhang auch denkbar, die Luftfeuchte der Druckluft in der Messgasleitung in Strömungsrichtung hinter der Luftbefeuchtung und/oder hinter dem Sensor zur Erfassung einer Eigenschaft der Druckluft zu messen, um sicherzustellen, dass jeweils zwar die erforderliche, aber keine zu hohe Luftfeuchtigkeit vorhanden ist.

Gemäß einer speziellen Ausführungsform der Erfindung ist die Ausgabeeinheit dazu eingerichtet, wenigstens eine Konzentration eines in der Druckluft enthaltenen Gases auf einer Anzeigeeinheit bzw. auf einem Display anzuzeigen. Im Übrigen ist es vorteilhaft, wenn die Ausgabeeinheit ferner dazu eingerichtet ist, bei Über- oder Unterschreitung eines Grenzwertes für eine Eigenschaft der Druckluft, insbesondere die Konzentration eines spezifischen Gases, ein Alarmsignal zu erzeugen. Gemäß einer weiteren speziellen Gestaltung der Ausgabeeinheit ist weiterhin eine Übertragungseinheit vorgesehen, sodass ein entsprechender Alarm an ein externes Gerät übertragbar ist. Eine derartige Übertragung kann sowohl drahtgebunden als auch drahtlos erfolgen. Hierbei ist es durchaus denkbar, dass ein derartiger Alarm an eine zentrale Überwachungseinrichtung, beispielsweise mit einem Alarmserver, oder an dezentral angeordnete Geräte, die von dem zuständigen Überwachungspersonal mitgeführt werden, übertragen wird.

Um eine möglichst kompakte Druckluftüberwachungseinrichtung bereitzustellen, ist ferner auf vorteilhafte Weise vorgesehen, dass der wenigstens eine Sensor zur Erfassung einer Eigenschaft, insbesondere einer Gaskonzentration, der Druckluft, der Luftbefeuchter und die Zapfstelle in einem Gehäuse angeordnet sind. Vorzugsweise handelt sich bei einem derartigen Gehäuse um einen Schrank, insbesondere einen Metallschrank, der mithilfe einer Tür sicher verschlossen werden kann. Hierbei ist es denkbar, dass in einem derartigen Gehäuse gleichfalls die erforderlichen elektronischen Komponenten zur Steuerung und Überwachung der Druckluftüberwachungseinrichtung vorgesehen sind. Ebenso ist es denkbar, dass diese Komponenten benachbart zu dem Gehäuse, in dem der Sensor zur Erfassung einer Eigenschaft der Druckluft, der Luftbefeuchter und die Zapfstelle vorgesehen sind, angeordnet sind.

Alternativ oder ergänzend zum Einsatz elektrochemischer Sensoren zur Erfassung einer Eigenschaft der Druckluft ist es ebenfalls möglich, Infrarotsensoren, die also auf einem optischen Messprinzip beruhen, einzusetzen. Generell ist die Erfindung nicht auf den Einsatz eines speziellen Sensortyps beschränkt. Vielmehr sind Sensoren, die unterschiedliche Messprinzipien nutzen, jeweils für den Einsatz in der erfindungsgemäßen Druckluftüberwachungseinrichtung einsetzbar, sofern sie auf geeignete Weise eine Eigenschaft der Druckluft, insbesondere die Konzentration eines in der Druckluft befindlichen Gases, erfassen können.

Auf vorteilhafte Weise ist mit dem beschriebenen System nicht nur die Überwachung der medizinischen Druckluft, insbesondere in einer Druckluftversorgungsanlage eines Krankenhauses, möglich, sondern darüber hinaus auch die Alarmierung sowie die lückenlose Dokumentation der Eigenschaften der Druckluft möglich.

Neben einer Druckluftüberwachungseinrichtung betrifft die Erfindung auch eine Drucklufterzeugungsanlage, die mit einer erfindungsgemäß ausgeführten Druckluftüberwachungseinrichtung kombiniert ist. Eine derartige Drucklufterzeugungsanlage für ein Krankenhaus umfasst hierbei einen Luftverdichter, der zum Beispiel als Hubkolbenkompressor oder Schraubenkompressor zum Verdichten von Umgebungsluft zu medizinischer Druckluft ausgeführt ist, eine Entfeuchtungseinrichtung zum Entfeuchten der angesaugten Umgebungsluft und der erfindungsgemäß ausgeführten Druckluftüberwachungseinrichtung zur Überwachung wenigstens eines Parameters der medizinischen Druckluft.

Auf bevorzugte Weise werden sämtliche durch die Druckluftüberwachungseinrichtung erzeugten Alarme an ein Alarmmanagementsystem übertragen, welches sowohl eine zentrale als auch eine dezentrale Alarmierung ermöglicht. Vorzugsweise werden die aufgenommenen Messparameter und/oder die jeweils hervorgerufenen Alarme fortlaufend dokumentiert, sodass nicht nur zu jeder Zeit eine Bereitstellung medizinischer Druckluft sichergestellt ist, sondern darüber hinaus auch frühzeitig Fehlentwicklungen innerhalb der Drucklufterzeugungs- bzw. Druckluftbereitstellungsanlage erkannt werden.

Im Folgenden wird die Erfindung ohne Beschränkung des allgemeinen Erfindungsgedankens anhand von Ausführungsbeispielen und unter Bezugnahme auf die Figuren näher erläutert. Dabei zeigen:
- Figur 1:: Druckluftüberwachungseinrichtung mit integrierter Zapfstelle, in die ein Stellorgan zur Einstellung des Volumenstroms eingesteckt ist sowie
- Figur 2:: Druckluftüberwachungseinrichtung mit integrierter Zapfstelle, in die kein Stellorgan zur Einstellung des Volumenstroms eingesteckt ist.

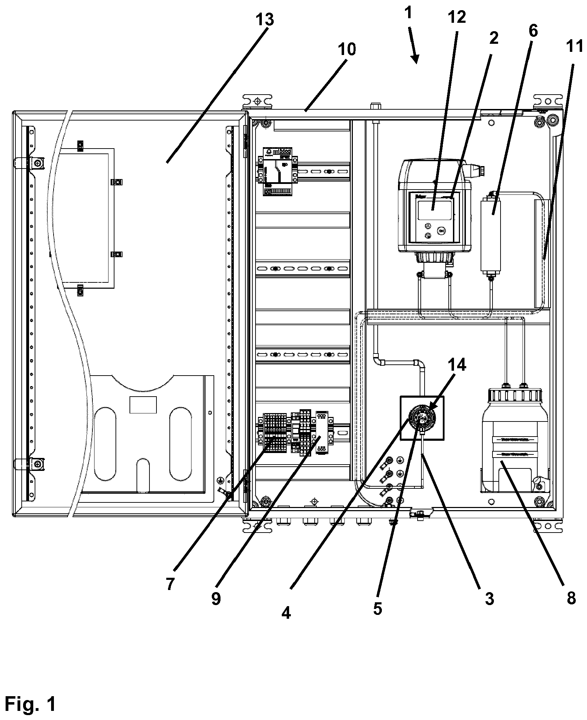

Figur 1 zeigt eine erfindungsgemäß ausgeführte Druckluftüberwachungseinrichtung 1, mit der die von einer Druckluftkonditionierungseinheit bereitgestellte medizinische Druckluft überwacht wird. Die Druckluftüberwachungseinrichtung 1 verfügt hierfür über einen Sensor 2 zur Erfassung einer Eigenschaft der Druckluft, der gemäß diesem Ausführungsbeispiel als elektrochemischen Sensor 2 ausgeführt ist, der die Konzentration von Kohlenstoffmonoxid (CO) in der von der Druckluftkonditionierungseinheit erzeugten Druckluft ermitteln kann. Die dargestellte Druckluftüberwachungseinrichtung 1 ist stromabwärts der Druckluftkonditionierungseinheit, die im Wesentlichen über einen Kompressor und einen Luftentfeuchter verfügt, angeordnet. Zwischen der Druckluftkonditionierungseinheit und der in Figur 1 gezeigten

Druckluftüberwachungseinrichtung 1 sind keine weiteren technischen Einrichtungen angeordnet.

Die Druckluftüberwachungseinrichtung 1 verfügt im Wesentlichen über eine Messluftleitung 3, über die Druckluft aus der Druckluftversorgungsanlage entnommen und schließlich dem elektrochemischen Sensor 2 zur Erfassung der Kohlenstoffmonoxidkonzentration in der Druckluft zugeführt wird.

Die von der Drucklufterzeugungsanlage in die Druckluftversorgungsleitungen eingeleitete medizinische Druckluft wird über einen Druckreduzierer auf einen Druck von beispielsweise 1,2 bar mit einem Volumenstrom von nicht mehr als etwa 1,0 l/min reduziert und in die Messluftleitung 3 geführt. Mit Hilfe des in eine als Druckluftkupplung 14 ausgeführte Zapfstelle 4 eingesteckten Stellorgans 5 wird zunächst der Volumenstrom innerhalb der Messluftleitung 3 auf einen Wert von 0,1 oder 0,2 l/min eingestellt. Eine Überwachung des in der Messluftleitung herrschenden Volumenstroms erfolgt mit Hilfe der Durchflussröhre 6. Sofern die Einstellung automatisiert vorgenommen wird, erfolgt dies unter Berücksichtigung eines in der Steuereinheit 7 hinterlegten Sollwerts sowie des von der Durchflussröhre 6 gemessenen Istwerts des Volumenstroms.

Wesentliche Komponente der Druckluftüberwachungseinrichtung 1 ist der CO-Sensor 2, mit dem die Konzentration an Kohlenstoffdioxid innerhalb der Druckluft erfasst wird. Um den CO-Sensor 2 nicht nachhaltig durch die Versorgung mit zu trockener Druckluft zu schädigen, ist dem CO-Sensor 2 in Strömungsrichtung ein Luftbefeuchter 8 vorgeschaltet.

Der Luftbefeuchter 8 weist ein fluiddichtes Gehäuse 10 auf. Innerhalb des Gehäuses 10 ist ein Schlauch aus einer semipermeablen wasserdurchlässigen Membran, beispielsweise aus Nafion, angeordnet. Der Schlauch stellt in diesem Bereich auch die Messluftleitung 3 dar.

Durch den Schlauch wird die medizinische Druckluft durch einen von dem Gehäuse des Luftbefeuchters 8 eingeschlossen Raum geleitet.

Innerhalb des Raumes befindet sich eine gesättigte Wasser-Salz-Lösung, wobei das Salz, bevorzugt Magnesiumchlorid, auf dem Boden liegt. Oberhalb der Wasser-Salz-Lösung befindet sich feuchte Luft mit einer konstanten relativen Luftfeuchtigkeit im Bereich von ungefähr 30 %.

Gemäß dem hier erläuterten Ausführungsbeispiel wird durch die Messluftleitung 3, und damit durch den Schlauch im Inneren des Luftbefeuchters 8, medizinische Druckluft mit einem Volumenstrom von 0,1 oder 0,2 l/min geleitet. Der Schlauch ist derart dimensioniert, dass dieser eine ausreichende Länge und Oberfläche, sodass die Feuchtigkeit der feuchten Luft durch den Schlauch in die medizinische Druckluft diffundiert und diese auf eine relative Luftfeuchtigkeit im Bereich zwischen 25 und 30 % angefeuchtet wird.

Nach dem Anfeuchten der medizinischen Druckluft wird diese über die Messluftleitung 3 weitergeleitet und zunächst durch die Durchflussröhre 6 geführt bevor sie dem CO-Sensor 2 zugeleitet wird. Auf diese Weise erhält der Sensor medizinische Druckluft mit einer konstanten relativen Luftfeuchtigkeit im Bereich zwischen 25 und 30 %, sodass dieser nicht austrocknet und zuverlässig über einen längeren Zeitraum, der bevorzugt länger als sechs Monate ist, eingesetzt werden kann. Ein häufiges Austauschen des CO-Sensors kann so zuverlässig vermieden werden.

Um den Volumenstrom innerhalb der Messgasleitung 3 auf den gewünschten Wert einstellen zu können, ist das Stellorgan 5 zur Einstellung des Volumenstroms vorgesehen. Dieses Stellorgan 5 verfügt über einen Anschluss, der derart ausgeführt ist, dass das Stellorgan 5 in eine herkömmliche Druckluftkupplung 14 für Druckluftverbraucher eingesteckt werden kann. Ein Werkzeug ist weder für das Einstecken noch das Entfernen des Stellorgans in bzw. aus der Zapfstelle erforderlich. In dem in Figur 1 gezeigten Ausführungsbeispiel befindet sich das Stellorgan 5 in eingesteckter Position in der als Druckluftkupplung 14 ausgeführten Zapfstelle 4.

Solange das Stellorgan 5 zur Einstellung des Volumenstroms in die Zapfstelle 4, eingesteckt ist, wird hierdurch ein konstanter Volumenstrom innerhalb der Messluftleitung 3 eingestellt. Mit dem Stellorgan 5, das über eine Blende verfügt, können unterschiedliche diskrete Volumenstromwerte von 0,1 l/min, 0,2 l/min, 0,3 l/min, 0,4 l/min, 0,5 l/min, 0,6 l/min, 0,8 l/min oder 1,0 l/min eingestellt werden. Bevorzugt wird am Stellorgan 5 ein Volumenstrom von 0,1 oder 0,2 l/min eingestellt. Eine Überwachung des jeweils eingestellten Sollwerts für den Volumenstrom erfolgt mit Hilfe der Durchflussröhre 6. Auf vorteilhafte Weise kann basierend auf den mit der Durchflussröhre 6 ermittelten Werten für den momentanen Volumenstrom eine manuelle oder automatisierte Verstellung des Stellorgans 5 vorgenommen werden. Weiterhin ist es denkbar, entsprechende Messwerte drahtgebunden oder bevorzugt drahtlos an eine zentrale Auswerteeinheit 9 zu übertragen. Darüber hinaus können die durch eine geeignete ausgeführte Durchflussröhre 6 erzeugten Messsignale verwendet werden, um mithilfe eines automatisiert verstellbaren Stellorgans 5, das eine Blende oder ein sonstiges Regelventil aufweist, den durch die Messluftleitung 3 zum CO-Sensor 2 strömenden Volumenstrom einzustellen.

Die in Figur 1 dargestellte Druckluftüberwachungseinrichtung 1 zur dauerhaften Überwachung des CO-Anteils von medizinischer Druckluft ist in kompakter Form innerhalb eines Gehäuses 10 angeordnet. In dem Gehäuse 10, das durch eine Tür 13 verschließbar ist, sind somit der CO Sensor 2, die Zapfstelle 4 mit dem darin eingesteckten Stellorgan 5, die Luftbefeuchtungseinheit 8 und Durchflussröhre 6, angeordnet. Weiterhin ist eine Beleuchtung 11 vorgesehen, um auch bei schlechten Lichtverhältnissen an der Druckluftüberwachungseinrichtung 1 Arbeiten ausführen, insbesondere Messwerte für die CO-Konzentration von einer Ausgabeeinheit 12 ablesen oder eine Druckluftprobe über die Zapfstelle 4 entnehmen zu können.

Mithilfe dieses Stellorgans 5 kann auf vorteilhafte Weise der Volumenstrom innerhalb der Messluftleitung eingestellt werden. Sofern die Durchflussröhre 6 regelungstechnisch an den Stellorgan 5 gekoppelt ist, kann der Volumenstrom in der Messluftleitung 3 in Abhängigkeit des von der Durchflussröhre 6 erzeugten Messsignals verändert werden. Generell kann der Automatisierungsgrad der Druckluftüberwachungseinrichtung 1 und auch die Art der Datenübertragung an die Gegebenheiten in dem jeweiligen Krankenhaus angepasst werden. In Abhängigkeit dieser Gegebenheiten bzw. der vorliegenden Anforderungen kann der CO-Sensor 2 über unterschiedliche Datenschnittstellen verfügen. In Abhängigkeit des Bedarfs kann entweder ein analoges Messsignal von 4 - 20 mA erzeugt werden oder die Signalübertragung erfolgt über ein BUS-System, wie etwa HART, LON, Profibus oder Foundation-Fieldbus.

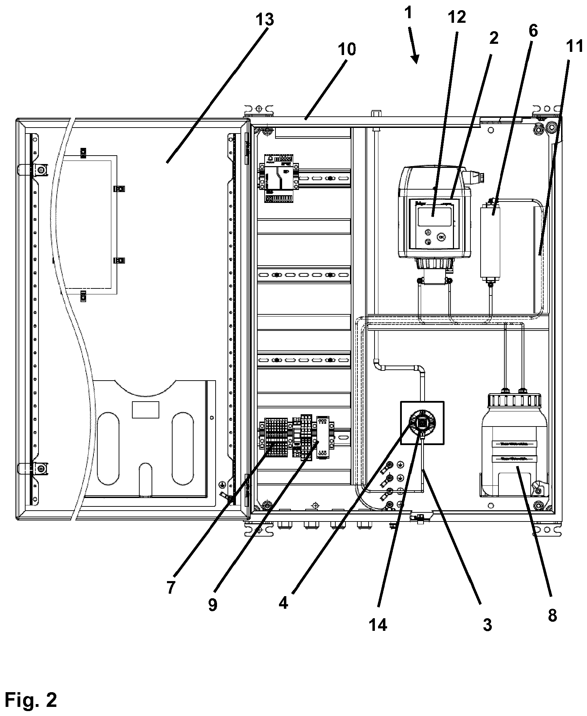

Figur 2 zeigt eine erfindungsgemäß ausgeführte Druckluftüberwachungseinrichtung 1, die über die gleichen Komponenten, wie sie bereits im Zusammenhang mit Figur 1 erläutert wurden, verfügt. Gleiche Komponenten sind mit gleichen Bezugszeichen gekennzeichnet. Wesentlich ist, dass ebenso wie in Figur 1, sämtliche Komponenten kompakt in einem verschließbaren Gehäuse 10 angeordnet sind. Im Gegensatz zum Betriebszustand, wie er in Figur 1 gezeigt wird, befindet sich in der Darstellung gemäß Figur 1 das Stellorgan 5 nicht innerhalb der als Druckluftkupplung ausgeführten Zapfstelle 4. Vielmehr ist die Druckluftkupplung in diesem Betriebszustand im Druckluftentnahmemodus frei zugänglich und kann als Zapfstelle 4 zur Entnahme einer Druckluftprobe aus der Druckluftanlage verwendet werden. Sobald die turnusmäßig durchgeführte Druckluftentnahme abgeschlossen ist, kann das Stellorgan 5 wiederum in die Druckluftkupplung eingesteckt werden, sodass abermals dauerhaft ein konstanter Volumenstrom entsprechend der Einstellung des Stellorgans 5 die Messluftleitung 3 durchströmt.

Die erfindungsgemäße technische Lösung ermöglicht somit auf verhältnismäßig einfache Weise die Kombination der Messung einer Kohlenmonoxidkonzentration in medizinischer Druckluft mit der Möglichkeit zur Entnahme einzelner Druckluftproben.

### BEZUGSZEICHENLISTE

- 1: Druckluftüberwachungseinrichtung
- 2: Sensor zur Erfassung einer Drucklufteigenschaft
- 3: Messluftleitung
- 4: Zapfstelle
- 5: Stellorgan
- 6: Durchflussröhre
- 7: Steuereinheit
- 8: Luftbefeuchter
- 9: Auswerteeinheit
- 10: Gehäuse
- 11: Beleuchtung
- 12: Ausgabeeinheit
- 13: Tür
- 14: Druckluftkupplung

## Patentansprüche

1. Überwachungseinrichtung (1) für eine Anlage zur Erzeugung medizinischer Druckluft mit einer Messluftleitung (3) zur Entnahme von Druckluft aus einer Druckluftversorgungsleitung stromabwärts einer Druckluftkonditionierungseinheit, mit wenigstens einem Sensor (2) zur Erzeugung eines Messsignals in Abhängigkeit einer Eigenschaft der durch die Messluftleitung (3) geförderten Druckluft, mit wenigstens einem Luftbefeuchter (8) zur Befeuchtung der Druckluft stromaufwärts des Sensors (2) und mit wenigstens einer Ausgabeeinheit (12), die unter Zugrundelegung des Messsignals eine Information über die Eigenschaft der Druckluft an einen Nutzer ausgibt,
**dadurch gekennzeichnet, dass** die Überwachungseinrichtung (1) eine Zapfstelle (4) zur Entnahme von Druckluft und ein Stellorgan (5) zur Veränderung eines in der Messluftleitung (3) herrschenden Volumen- und/oder Massenstromes der Druckluft umfasst, wobei das Stellorgan (5) in einem Messmodus in die Zapfstelle (4) eingesteckt und in einem Druckluftentnahmemodus aus der Zapfstelle entnommen ist und
wobei in dem Druckluftentnahmemodus eine Druckluftprobe über die Zapfstelle entnehmbar ist.

2. Überwachungseinrichtung (1) nach Anspruch 1,
**dadurch gekennzeichnet, dass** das Stellorgan eingerichtet ist, verschiedene diskrete Werte des Volumen- und/oder Massenstroms in der Messluftleitung (3) einzustellen.

3. Überwachungseinrichtung (1) nach Anspruch 1 oder 2,
**dadurch gekennzeichnet, dass** das Stellorgan (5) dazu eingerichtet ist, zumindest Volumenströme von 1,67 . 10⁻⁶ m³/s (0,1 l/min) oder 3,33 · 10⁻⁶ m³/s (0,2 l/min) einzustellen.

4. Überwachungseinrichtung (1) nach einem der Ansprüche 1 bis 3,
**dadurch gekennzeichnet, dass** das Stellorgan (5) wenigstens eine Blende aufweist.

5. Überwachungseinrichtung (1) nach einem der Ansprüche 1 bis 4,
**dadurch gekennzeichnet, dass** das Stellorgan (5) zur Einstellung des in der Messluftleitung (3) herrschenden Volumenstroms und/oder Massenstromes zerstörungsfrei und/oder werkzeugfrei in die Zapfstelle (4) einsteckbar und aus der Zapfstelle (4) entnehmbar ist.

6. Überwachungseinrichtung (1) nach einem der Ansprüche 1 bis 5,
**dadurch gekennzeichnet, dass** der wenigstens eine Sensor (2) zur Erfassung wenigstens einer Eigenschaft der Druckluft als elektrochemischer Sensor ausgeführt ist.

7. Überwachungseinrichtung (1) nach einem der Ansprüche 1 bis 6,
**dadurch gekennzeichnet, dass** der zumindest eine Sensor (2) zur Erfassung wenigstens einer Eigenschaft der Druckluft als CO-Sensor ausgeführt ist, der in Abhängigkeit des CO-Gehaltes der Druckluft das Messsignal erzeugt.

8. Überwachungseinrichtung (1) nach einem der Ansprüche 1 bis 7,
**dadurch gekennzeichnet ist, dass** die Ausgabeeinheit (12) dazu eingerichtet ist, wenigstens eine Konzentration eines Gases in der Druckluft auf einer Ausgabeeinheit (12) anzuzeigen.

9. Überwachungseinrichtung (1) nach einem der Ansprüche 1 bis 8,
**dadurch gekennzeichnet, dass** die Ausgabeeinheit (12) dazu eingerichtet ist, bei Über- oder Unterschreitung eines Grenzwertes für eine Eigenschaft der Druckluft ein Alarmsignal zu erzeugen.

10. Überwachungseinrichtung (1) nach einem der Ansprüche 1 bis 9,
**dadurch gekennzeichnet, dass** die Ausgabeeinheit (12) eine Übertragungseinheit aufweist, über die die eine Information über eine Eigenschaft der Druckluft an ein externes Gerät übertragbar ist.

11. Überwachungseinrichtung (1) nach einem der Ansprüche 1 bis 10,
**dadurch gekennzeichnet, dass** wenigstens ein Sensor (2) zur Erfassung wenigstens einer Eigenschaft der Druckluft, der Luftbefeuchter (8) und die Zapfstelle (4) in einem Gehäuse (10) angeordnet sind.

12. Überwachungseinrichtung (1) nach Anspruch 11,
**dadurch gekennzeichnet, dass** in dem Gehäuse (10) wenigstens eine Beleuchtungseinheit (11) angeordnet ist.

13. Überwachungseinrichtung (1) nach Anspruch 11 oder 12,
**dadurch gekennzeichnet, dass** in dem Gehäuse (10) eine Steuereinheit (7) und/oder eine Auswerteeinheit (9) angeordnet sind.

14. Überwachungseinrichtung (1) nach einem der Ansprüche 1 bis 13,
**dadurch gekennzeichnet, dass** als Sensor (2) zur Erfassung einer Eigenschaft der Druckluft ausschließlich ein Sensor zur Erfassung der Kohlenstoffmonoxidkonzentration vorgesehen ist.

15. Überwachungseinrichtung (1) nach einem der Ansprüche 1 bis 14,
**dadurch gekennzeichnet, dass** eine Durchflussröhre (6) zur Messung des in der Messluftleitung (3) herrschenden Volumen- und/oder Massenstroms vorgesehen ist.

16. Anlage zur Erzeugung und Verteilung von medizinischer Druckluft mit einer Überwachungseinrichtung (1) nach einem der vorangehenden Ansprüche.

## Claims

1. Monitoring device (1) for an apparatus for producing medical compressed air, comprising a measurement air conduit (3) for extracting compressed air from a compressed air supply conduit downstream of a compressed air conditioning unit, comprising at least one sensor (2) for producing a measurement signal on the basis of a property of the compressed air conveyed through the measurement air conduit (3), comprising at least one humidifier (8) for moistening the compressed air upstream of the sensor (2) and comprising at least one output unit (12) which outputs information about the property of the compressed air to a user on the basis of the measurement signal, **characterized in that** the monitoring device (1) comprises a tap (4) for extracting compressed air and an operating member (5) for altering a prevalent volumetric and/or mass flow rate of the compressed air in the measurement air conduit (3), the operating member (5) being inserted into the tap (4) in a measuring mode and being extracted from the tap in a compressed air extraction mode and
a compressed air sample being able to be extracted via the tap in the compressed air extraction mode.

2. Monitoring device (1) according to Claim 1,
**characterized in that** the operating member is configured to set different discrete values of the volumetric and/or mass flow rate in the measurement air conduit (3).

3. Monitoring device (1) according to Claim 1 or 2,
**characterized in that** the operating member (5) is configured to at least set volumetric flow rates of 1.67·10⁻⁶ m³/s (0.1 I/min) or 3.33·10⁻⁶ m³/s (0.2 l/min).

4. Monitoring device (1) according to any one of Claims 1 to 3, **characterized in that** the operating member (5) comprises at least one orifice plate.

5. Monitoring device (1) according to any one of Claims 1 to 4, **characterized in that** the operating member (5) is insertable into the tap (4) and extractable from the tap (4) in non-destructive fashion and/or without tools for the purposes of setting the prevalent volumetric flow rate and/or mass flow rate in the measurement air conduit (3).

6. Monitoring device (1) according to any one of Claims 1 to 5, **characterized in that** the at least one sensor (2) for registering at least one property of the compressed air is embodied as an electrochemical sensor.

7. Monitoring device (1) according to any one of Claims 1 to 6, **characterized in that** the at least one sensor (2) for registering at least one property of the compressed air is embodied as a CO sensor which produces the measurement signal on the basis of the CO content of the compressed air.

8. Monitoring device (1) according to any one of Claims 1 to 7, **characterized in that** the output unit (12) is configured to indicate on an output unit (12) at least a concentration of a gas in the compressed air.

9. Monitoring device (1) according to any one of Claims 1 to 8, **characterized in that** the output unit (12) is configured to produce an alarm signal if a limit for a property of the compressed air is overshot or undershot.

10. Monitoring device (1) according to any one of Claims 1 to 9, **characterized in that** the output unit (12) comprises a transfer unit, by means of which the information about a property of the compressed air is transferable to external equipment.

11. Monitoring device (1) according to any one of Claims 1 to 10, **characterized in that** at least a sensor (2) for registering at least one property of the compressed air, the humidifier (8) and the tap (4) are arranged within a housing (10).

12. Monitoring device (1) according to Claim 11,
**characterized in that** at least one lighting unit (11) is arranged within the housing (10).

13. Monitoring device (1) according to Claim 11 or 12,
**characterized in that** a control unit (7) and/or an evaluation unit (9) are arranged within the housing (10).

14. Monitoring device (1) according to any one of Claims 1 to 13, **characterized in that** solely a sensor for registering the carbon monoxide concentration is provided as a sensor (2) for registering a property of the compressed air.

15. Monitoring device (1) according to any one of Claims 1 to 14, **characterized in that** a flow tube (6) is provided for measuring the prevalent volumetric and/or mass flow rate in the measurement air conduit (3).

16. Apparatus for producing and distributing medical compressed air, comprising a monitoring device (1) according to any one of the preceding claims.

## Revendications

1. Dispositif de surveillance (1) pour une installation de production d'air comprimé médical, comprenant une conduite d'air de mesure (3) servant à prélever de l'air comprimé d'une conduite d'alimentation en air comprimé en aval d'une unité de conditionnement d'air comprimé, au moins un capteur (2) servant à produire un signal de mesure en fonction d'une propriété de l'air comprimé acheminé à travers la conduite d'air de mesure (3), au moins un humidificateur d'air (8) servant à humidifier l'air comprimé en aval du capteur (2), et au moins une unité de sortie (12) qui fournit à un utilisateur une information concernant la propriété de l'air comprimé, sur la base du signal de mesure,
**caractérisé en ce que** le dispositif de surveillance (1) comprend un point de prise (4) pour le prélèvement d'air comprimé et un élément de réglage (5) pour la modification d'un débit volumique et/ou massique de l'air comprimé régnant dans la conduite d'air de mesure (3), dans lequel l'élément de réglage (5) dans un mode de mesure est inséré dans le point de prise (4), et dans un mode de prélèvement d'air comprimé est sorti du point de prise, et dans lequel, dans le mode de prélèvement d'air comprimé, un échantillon d'air comprimé peut être prélevé par l'intermédiaire du point de prise.

2. Dispositif de surveillance (1) selon la revendication 1, **caractérisé en ce que** l'élément de réglage est conçu pour régler différentes valeurs discrètes du débit volumique et/ou massique dans la conduite d'air de mesure (3).

3. Dispositif de surveillance (1) selon la revendication 1 ou 2, **caractérisé en ce que** l'élément de réglage (5) est conçu pour régler au moins des débits volumiques de 1,67 • 10⁻⁶ m³/s (0,1 I/min) ou de 3,33 • 10⁻⁶ m³/s (0,2 l/min).

4. Dispositif de surveillance (1) selon l'une quelconque des revendications 1 à 3, **caractérisé en ce que** l'élément de réglage (5) présente au moins un obturateur.

5. Dispositif de surveillance (1) selon l'une quelconque des revendications 1 à 4, **caractérisé en ce que** pour le réglage du débit volumique et/ou du débit massique régnant dans la conduite d'air de mesure (3), l'élément de réglage (5) peut être inséré dans le point de prise (4) et retiré du point de prise (4) sans destruction et/ou sans outil.

6. Dispositif de surveillance (1) selon l'une quelconque des revendications 1 à 5, **caractérisé en ce que** ledit au moins un capteur (2) servant à détecter au moins une propriété de l'air comprimé est réalisé sous forme de capteur électrochimique.

7. Dispositif de surveillance (1) selon l'une quelconque des revendications 1 à 6, **caractérisé en ce que** ledit au moins un capteur (2) servant à détecter au moins une propriété de l'air comprimé est réalisé sous forme de capteur de CO qui produit le signal de mesure en fonction de la teneur en CO de l'air comprimé.

8. Dispositif de surveillance (1) selon l'une quelconque des revendications 1 à 7, **caractérisé en ce que** l'unité de sortie (12) est conçue pour afficher au moins une concentration d'un gaz dans l'air comprimé sur une unité de sortie (12).

9. Dispositif de surveillance (1) selon l'une quelconque des revendications 1 à 8, **caractérisé en ce que** l'unité de sortie (12) est conçue pour produire un signal d'alarme en cas de dépassement ou de soupassement d'une valeur limite pour une propriété de l'air comprimé.

10. Dispositif de surveillance (1) selon l'une quelconque des revendications 1 à 9, **caractérisé en ce que** l'unité de sortie (12) présente une unité de transmission qui permet de transmettre à un appareil externe une information concernant une propriété de l'air comprimé.

11. Dispositif de surveillance (1) selon l'une quelconque des revendications 1 à 10, **caractérisé en ce qu'**au moins un capteur (2) servant à détecter au moins une propriété de l'air comprimé, l'humidificateur d'air (8) et le point de prise (4) sont disposés dans un boîtier (10).

12. Dispositif de surveillance (1) selon la revendication 11, **caractérisé en ce qu'**au moins une unité d'éclairage (11) est disposée dans le boîtier (10).

13. Dispositif de surveillance (1) selon la revendication 11 ou 12, **caractérisé en ce qu'**une unité de commande (7) et/ou une unité d'évaluation (9) est/sont disposée(s) dans le boîtier (10).

14. Dispositif de surveillance (1) selon l'une quelconque des revendications 1 à 13, **caractérisé en ce que** seul un capteur servant à détecter la concentration en monoxyde de carbone est prévu comme capteur (2) servant à détecter une propriété de l'air comprimé.

15. Dispositif de surveillance (1) selon l'une quelconque des revendications 1 à 14, **caractérisé en ce qu'**un tube de passage (6) est prévu pour mesurer le débit volumique et/ou massique régnant dans la conduite d'air de mesure (3).

16. Installation de production et de distribution d'air comprimé médical, comprenant un dispositif de surveillance (1) selon l'une quelconque des revendications précédentes.
